# FASCICULE DE BREVET EUROPEEN

(11) **EP 3 753 538 B1**
(45) Date de publication et mention de la délivrance du brevet: **27.04.2022**
(21) Numéro de dépôt: 20181230.2
(22) Date de dépôt: 19.06.2020
(51) Int. Cl.: A61F 7/02, A61H 9/00, A61F 5/34, A61F 7/10

(54) **ORTHESE ADAPTEE POUR LE TRAITEMENT PAR CRYOTHERAPIE ET/OU PRESSOTHERAPIE D'UNE ZONE D'UN CORPS HUMAIN OU ANIMAL ET SON PROCEDE DE FABRICATION**
ORTHESE FÜR DIE BEHANDLUNG MIT KRYOTHERAPIE UND/ODER PRESSOTHERAPIE EINER KÖRPERZONE VON MENSCH ODER TIER, UND IHR HERSTELLUNGSVERFAHREN
ORTHOSIS ADAPTED FOR TREATMENT BY CRYOTHERAPY AND/OR PRESSOTHERAPY OF AN AREA OF A HUMAN OR ANIMAL BODY AND METHOD FOR MANUFACTURING SAME

(30) Priorité: 20.06.2019 FR 1906689
(43) Date de publication de la demande: 23.12.2020
(73) Titulaire: Carabalona, Cédric, 13840 Rognes (FR)
(72) Inventeur: Carabalona, Cédric, 13840 Rognes (FR)
(74) Mandataire: Roman, Alexis

(56) Documents cités:
- WO-A1-95/03016
- WO-A1-98/48759
- WO-A1-2014/138790
- WO-A1-2014/184324
- WO-A1-2015/084925
- WO-A2-02/058604
- US-A1- 2005 256 556
- US-A1- 2015 305 960

## Description

### Domaine technique.

L'invention a pour objet une orthèse adaptée pour le traitement par cryothérapie et/ou pressothérapie d'une zone d'un corps humain ou animal. Elle concerne également un procédé de fabrication de cette orthèse.

L'invention se rapporte au domaine technique des dispositifs de traitement par cryothérapie et/ou pressothérapie et plus particulièrement des dispositifs de traitement pour les membres du corps humain ou animal.

### État de la technique.

La cryothérapie est une technique de traitement par le froid bien connue notamment utilisée pour diminuer un oedème ou un gonflement, réduire la douleur, soulager une inflammation notamment dues à des lésions, permettre une récupération musculaire évitant ainsi les courbatures et contractures musculaires, etc. Cette technique convient parfaitement pour la préparation sportive, la récupération physique ou encore pour améliorer les performances physiques. L'utilisation rapide de cette technique peut également permettre de réduire les saignements primaires grâce à un effet vasoconstricteur.

La pressothérapie est une technique de traitement par pression active bien connue notamment pour éliminer les gonflements et oedèmes présents dans la zone traitée. Cette technique reproduit la contraction naturelle des muscles. La pressothérapie se décline en pressothérapie avec effet pompage et en pressothérapie avec effet drainage.

La pressothérapie avec effet pompage est une technique de compression pneumatique par intermittence qui par une action de gonflage et dégonflage sur une zone du corps reproduit la contraction naturelle des muscles.

La pressothérapie avec effet drainage est une technique de traitement par pression bien connue notamment pour aider à l'amélioration de la circulation de la lymphe. Cette technique permet d'améliorer l'élimination des toxines accumulées dans les cellules et de diminuer les œdèmes en vue d'améliorer la récupération physique et musculaire.

L'utilisation des techniques de cryothérapie et de pressothérapie est la plupart du temps réalisée de manière séparée. Il existe des orthèses composées d'une enveloppe pour la pressothérapie et d'une enveloppe pour la cryothérapie, mais elles fonctionnent alternativement, et non pas simultanément. Les documents brevet WO 2015/084925 (CoolSystems) ou US2005/0256556 (CoolSystems) divulguent par exemple des orthèses comportant une enveloppe pour la cryothérapie et une enveloppe pour la pressothérapie avec effet pompage. Ces orthèses ne sont toutefois pas adaptées pour un traitement en profondeur du membre, et en pratique, offrent une gamme de traitements thérapeutiques relativement limités.

Un objectif de l'invention est de remédier aux inconvénients précités.

Un autre objectif de l'invention est d'améliorer les orthèses existantes pour offrir à l'utilisateur une gamme de traitements thérapeutiques plus larges.

Encore un autre objectif de l'invention est de proposer un procédé de fabrication d'une orthèse qui soit simple, rapide et peu onéreux.

### Présentation de l'invention.

La solution proposée par l'invention est une orthèse adaptée pour un traitement par cryothérapie et/ou pressothérapie d'une zone d'un corps humain ou animal comportant : une première enveloppe adaptée pour un traitement par cryothérapie, laquelle première enveloppe comporte une poche adaptée pour recevoir un liquide et une deuxième enveloppe adaptée pour un traitement par pressothérapie avec effet de pompage, laquelle deuxième enveloppe comporte une seule poche gonflable. L'orthèse est remarquable en ce qu'elle comporte en outre une troisième enveloppe adaptée pour un traitement par pressothérapie avec effet de drainage, laquelle troisième enveloppe comporte plusieurs poches gonflables indépendantes.

L'orthèse comportant ces trois enveloppes permet de traiter par cryothérapie et par pressothérapie de manière simultanée. Les enveloppes de pressothérapie selon l'invention permettent d'obtenir un effet de pompage et un effet de drainage qui peuvent fonctionner séparément, simultanément ou en combinaison avec le traitement par cryothérapie. L'invention permet ainsi d'obtenir une gamme plus étendue de traitements, mais également un traitement plus efficace.

D'autres caractéristiques avantageuses de l'invention sont listées ci-dessous. Chacune de ces caractéristiques peut être considérée seule ou en combinaison avec les caractéristiques remarquables définies ci-dessus, et faire l'objet, le cas échéant, d'une ou plusieurs demandes de brevet divisionnaires :
- Selon un mode de réalisation, la première enveloppe est formée d'une première couche et d'une deuxième couche assemblées par soudure de leurs bords.
- Selon un mode de réalisation, la troisième enveloppe est formée d'une troisième et d'une quatrième couche assemblées par soudure de leurs bords.
- Selon un mode de réalisation, la première enveloppe et la troisième enveloppe sont assemblées par soudure.
- Selon un mode de réalisation, la deuxième enveloppe est formée par soudure de la première enveloppe et de la troisième enveloppe.
- Selon un mode de réalisation, les poches indépendantes sont formées par soudure de la troisième couche et de la quatrième couche.
- Selon un mode de réalisation, chaque poche indépendante est reliée à une valve.
- Selon un mode de réalisation, l'orthèse comporte un revêtement textile.
- Selon un mode de réalisation, un revêtement calorifuge est disposé entre le revêtement textile et la troisième enveloppe.

Un autre aspect de l'invention concerne un procédé de fabrication d'une orthèse conforme à l'une des caractéristiques précédentes, comportant les étapes consistant à :
- former la première enveloppe en assemblant une première couche d'un matériau étanche (au moins au liquide) et une deuxième couche d'un matériau étanche (au gaz et au liquide), de manière à délimiter la poche hydraulique,
- former la troisième enveloppe en assemblant une troisième couche d'un matériau étanche (au moins au gaz) et une quatrième couche d'un matériau étanche (au moins au gaz), de manière à délimiter les poches gonflables indépendantes,
- former la deuxième enveloppe en assemblant la première enveloppe et la troisième enveloppe de sorte que la deuxième couche et la troisième couche délimitent la poche de ladite deuxième enveloppe.

Selon un mode de réalisation, le revêtement calorifuge est cousu sur la quatrième couche.

Selon un mode de réalisation, le revêtement textile vient recouvrir le revêtement calorifuge.

Selon un mode de réalisation, le revêtement textile vient recouvrir le première couche.

Encore un autre aspect de l'invention concerne un ensemble comportant l'orthèse et une hipposandale pour le traitement des pattes d'animaux, ladite hipposandale comprenant une semelle cryogénique, une partie textile et un tube vortex.

Selon un mode de réalisation, la semelle cryogénique comporte une demi-coquille inférieure et une demi-coquille supérieure.

Selon un mode de réalisation, la demi-coquille supérieure comporte au moins une perforation.

Selon un mode de réalisation, le tube vortex est relié à la semelle cryogénique.

Selon un mode de réalisation, le textile est relié à la semelle cryogénique de manière à pouvoir envelopper un sabot.

### Brève description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
[Fig. 1] est une vue schématique d'une orthèse selon l'invention, montrant les différentes enveloppes,
[Fig. 2] est une vue en coupe d'une orthèse selon l'invention, montrant les différentes couches selon un premier mode de réalisation,
[Fig. 3] est une vue en gros plan d'une valve,
[Fig. 4] est une vue en coupe d'une orthèse selon l'invention, montrant les différentes couches selon un deuxième mode de réalisation,
[Fig. 5] est une vue en coupe d'une orthèse selon l'invention montrant les différentes couches selon un troisième mode de réalisation comportant une couche supplémentaire,
[Fig. 6a] illustre une première étape d'un procédé de fabrication de l'orthèse selon le premier mode de réalisation,
[Fig. 6b] illustre une deuxième étape d'un procédé de fabrication de l'orthèse selon le premier mode de réalisation,
[Fig. 6c] illustre une troisième étape d'un procédé de fabrication de l'orthèse selon le premier mode de réalisation,
[Fig. 6d] illustre une quatrième étape d'un procédé de fabrication de l'orthèse selon le premier mode de réalisation,
[Fig. 6e] illustre une cinquième étape d'un procédé de fabrication de l'orthèse selon le premier mode de réalisation,
[Fig. 6f] illustre une sixième étape d'un procédé de fabrication de l'orthèse selon un premier mode de réalisation,
[Fig. 7a] illustre une première étape d'un procédé de fabrication de l'orthèse selon le troisième mode de réalisation,
[Fig. 7b] illustre une deuxième étape d'un procédé de fabrication de l'orthèse selon le troisième mode de réalisation,
[Fig. 7c] illustre une troisième étape d'un procédé de fabrication de l'orthèse selon le troisième mode de réalisation,
[Fig. 7d] illustre une quatrième étape d'un procédé de fabrication de l'orthèse selon le troisième mode de réalisation,
[Fig. 7e] illustre une cinquième étape d'un procédé de fabrication de l'orthèse selon le troisième mode de réalisation,
[Fig. 7f] illustre une sixième étape d'un procédé de fabrication de l'orthèse selon le troisième mode de réalisation,
[Fig. 7g] illustre une septième étape d'un procédé de fabrication de l'orthèse selon le troisième mode de réalisation,
[Fig. 8a] est une représentation d'un deuxième mode de réalisation du déplacement du gaz dans une troisième enveloppe,
[Fig. 8b] est une représentation d'un troisième mode de réalisation du déplacement du gaz dans une troisième enveloppe,
[Fig. 9] est une vue de profil d'une hipposandale avec traitement cryogénique selon un premier mode de réalisation.
[Fig. 10] est une vue de profil d'une hipposandale avec traitement cryogénique selon un second mode de réalisation.

### Description des modes de réalisation.

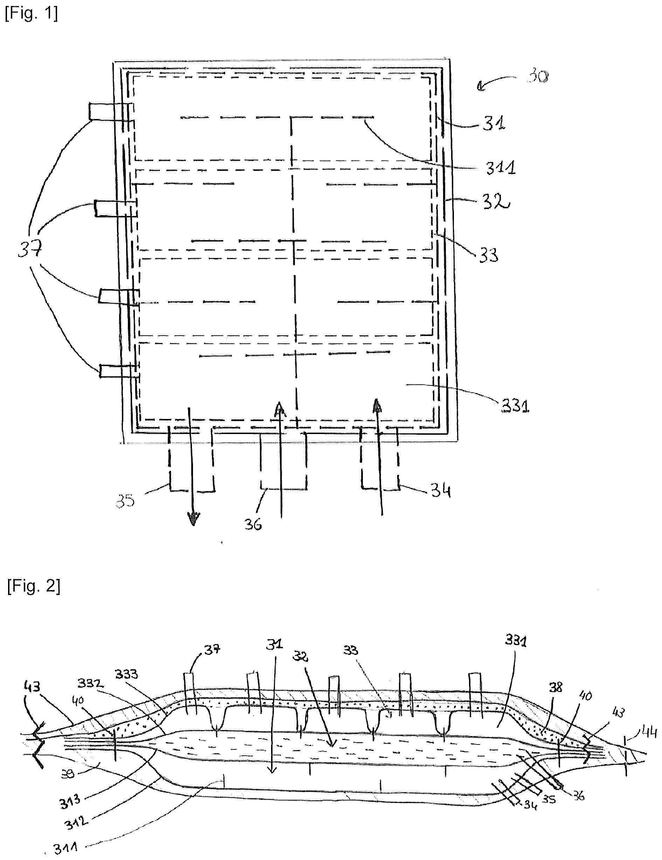

La figure 1 représente une vue schématique de l'orthèse 30 selon l'invention. L'orthèse 30 comporte une première enveloppe 31 adaptée pour le traitement par cryothérapie. Ladite première enveloppe 31 est formée par assemblage d'une première couche 312 et d'une deuxième couche 313 afin de former une poche unique contenant le circuit hydraulique 311. Lors de l'assemblage de la première couche 312 et de la deuxième couche 313, un circuit hydraulique 311 est formé par soudure de la première couche 312 et de la deuxième couche 313 pour délimiter le parcours du fluide hydraulique. Ladite première enveloppe 31 est reliée à une valve d'entrée 34 adaptée pour être connectée au circuit hydraulique 311 d'une machine de cryothérapie et à une valve de sortie 35 d'une machine de pressothérapie. Le fluide hydraulique rentre dans la première enveloppe 31 par la valve d'entrée 34, circule dans ladite enveloppe 31 en suivant le circuit hydraulique 311 jusqu'à la sortie de ladite enveloppe 31 par la valve de sortie 35. Le fluide hydraulique en suivant le cheminement défini par le circuit hydraulique 311 se déplacera de manière à rendre optimal le traitement par cryothérapie. Ladite première enveloppe 31 permet de refroidir le membre afin d'augmenter la récupération ou la préparation des muscles du membre traité. Le fluide utilisé est préférentiellement de l'eau déminéralisée mélangée à un antigel. Le volume de l'antigel peut varier de 20% à 50%. Le débit du fluide est compris entre 1 L/min et 15 L/min sous une pression comprise entre 0,1 bar et 4 bars.

L'orthèse 30 comporte également une troisième enveloppe 33 adaptée pour le traitement par pressothérapie avec effet de drainage. Ladite troisième enveloppe 33 est formée par assemblage d'une troisième couche 332 et d'une quatrième couche 333. Ladite troisième enveloppe 33 est compartimentée en plusieurs poches 331 indépendantes les unes des autres. Chaque poche 331 est reliée à une valve d'entrée 37 permettant de faire entrer et sortir un gaz sous pression dans ladite poche 331. Le gaz utilisé est préférentiellement de l'air. Le débit d'air est compris entre 15 L/min et 100 L/min sous une pression comprise entre 0,05 bar et 1 bar. On peut ainsi gonfler séparément les poches 331 afin d'obtenir un effet de vague se déplaçant progressivement le long du membre à traiter.

L'orthèse 30 comporte également une deuxième enveloppe 32 adaptée pour le traitement par pressothérapie avec effet de pompage afin de former une poche unique pour le pompage. Ladite deuxième enveloppe 32 est reliée à une valve d'entrée pneumatique 36 permettant de contrôler l'entrée et la sortie d'un gaz sous pression afin d'adapter le gonflement de la poche en fonction du traitement par pressothérapie avec effet de pompage que l'on souhaite effectuer. Ladite deuxième enveloppe 32 est formée par assemblage de la première enveloppe 31 et de la troisième enveloppe 33. La poche de la deuxième enveloppe 32 est délimitée par la deuxième couche 313 et la troisième couche 332.

Les première 312, deuxième 313, troisième 332 et quatrième couches 333 de l'orthèse 30 sont de formes variables afin de s'adapter aux différents membres sur lesquels elles doivent être placées. Elles peuvent être rondes, carrées, rectangulaires, trapézoïdales, polygonales, etc. La liste des formes énoncées n'est pas exhaustive, car il peut exister autant de formes que d'adaptations aux membres humain ou animal à traiter.

Les différentes couches 312, 313, 332 et 333 permettent la formation des différentes enveloppes de manière à ce que les enveloppes soient étanches au gaz et au fluide frigorifique.

Les différentes couches 312, 313, 332 et 333 sont de dimensions variables par exemple d'une longueur comprise entre 20 cm et 150 cm une largeur comprise entre 15 cm et 70 cm. Ces deux exemples ne sont pas limitatifs et les dimensions de l'orthèse 30 peuvent être adaptées en fonction de la taille du membre humain ou animal à traiter.

Les différentes couches 312, 313, 332 et 333 ont une épaisseur pouvant varier entre 0,1 mm et 25 mm.

Le matériau utilisé pour lesdites première couche 312 et deuxième couche 313 est préférentiellement un matériau étanche au gaz et au liquide. On utilise avantageusement du polyuréthane, car il est résistant à l'humidité et constitue un bon isolant thermique et c'est un matériau peu onéreux.

La figure 2 est une vue en coupe d'une orthèse 30 qui représente le positionnement des différentes enveloppes les unes par rapport aux autres. La description suivante présente l'enchainement des couches, en allant du corps du patient vers l'extérieur.

Les bords de la première couche 312 et de la deuxième couche 313 sont assemblés entre eux par soudure 40 de manière à former la poche hydraulique de la première enveloppe 31. Le circuit hydraulique 311 est également formé à cette étape par soudure de la première couche 312 et de la deuxième couche 313. Une valve d'entrée 34 hydraulique et une valve de sortie 35 hydraulique sont soudées à cette première enveloppe 31. La première enveloppe 31 est destinée à être placée contre le corps du patient.

Les bords de la troisième couche 332 et de la quatrième couche 333 sont assemblés entre eux par soudure 40 de manière à former la poche de drainage de la troisième enveloppe 33. Sur la figure 2, la troisième enveloppe 33 est disposée dans l'orthèse 30 à l'opposé de la première enveloppe 31. Ladite troisième enveloppe 33 est compartimentée en plusieurs poches 331 indépendantes, ce compartimentage étant réalisé par soudure 40 de la troisième couche 332 et de la quatrième couche 333. Les poches 331 ne communiquent pas entre elles. Chaque dite poche 331 est reliée à une valve d'entrée 37. La troisième enveloppe 33 peut donc se retrouver dans différents états. Un état dans lequel toutes les poches 331 sont dégonflées. Un autre état dans lequel l'ensemble des poches 331 sont gonflées. Un autre état dans lequel une partie seulement des poches 331 est gonflée et l'autre partie desdites poches 331 est dégonflée. Ces différents états permettent de créer un effet de vague sur le membre à traiter par effet de compression et décompression réalisée de manière locale et séquencée. Ceci est une liste non limitative des différents états que peut présenter la troisième enveloppe 33.

Dans un exemple où la troisième enveloppe 33 serait composée de trois poches 331 indépendantes, le drainage avec effet de vague fonctionnerait comme suit. On gonfle la première poche, puis la deuxième poche (en maintenant la première poche gonflée), puis la troisième poche (en maintenant la première poche et la deuxième poche gonflées). On peut ensuite dégonfler la troisième poche (en maintenant la première poche et la deuxième poche gonflées), puis dégonfler la deuxième poche (en maintenant la première poche gonflée et la troisième poche dégonflée), puis éventuellement dégonfler la première poche (en maintenant la deuxième poche dégonflée et la troisième poche dégonflée). Et ainsi de suite pour obtenir un effet de vagie qui se déplace le long de tout ou partie de la zone à traiter.

On peut encore par exemple gonfler la première poche, puis la dégonfler, puis gonfler la deuxième poche (en maintenant la première poche et la troisième poche dégonflées) et la dégonfler, puis gonfler la troisième poche (en maintenant la première poche et la deuxième poche dégonflées) et la dégonfler. Et ainsi de suite pour obtenir un effet de vague qui avance le long de tout ou partie de la zone à traiter.

Sur la figure 2, la deuxième enveloppe 32 est disposée entre les deux dites enveloppes précédentes 31, 33. La poche de ladite deuxième enveloppe 32 est formée par soudure 40 des bords de la troisième enveloppe 33 et des bords de la première enveloppe 31. Ladite deuxième enveloppe 32 est reliée à la valve pneumatique 36 permettant l'entrée et la sortie du gaz. Ladite deuxième enveloppe 32 est composée d'une poche unique. Ladite deuxième enveloppe 32 peut donc se retrouver dans différents états. Un état dans lequel la poche unique est gonflée. Un autre état dans lequel la poche unique est dégonflée. Un autre état encore dans lequel la poche unique est en phase de gonflage ou en phase de dégonflage. Pour obtenir l'effet de pompage, on peut alterner les phases de gonflage et de dégonflage de la poche avec par exemple une alternance comprise entre 1 s et 30 s, en suivant par exemple une courbe sinusoïdale. Ces différents états de l'enveloppe permettent une compression et une décompression totale de la zone à traiter du membre et une amélioration de la circulation sanguine et/ou lymphatique. Ceci est une liste non limitative des différents états que peut présenter la deuxième enveloppe 32.

Un revêtement calorifuge 38 est avantageusement placé contre la quatrième couche 333 de la troisième enveloppe 33 afin de faciliter son assemblage. Le revêtement calorifuge 38 assure une isolation thermique permettant de retenir le froid pour augmenter le rendement de la thérapie cryogénique. On utilise préférentiellement une couche d'aluminium, car c'est un matériau léger, très résistant, étanche et extrêmement souple. Le revêtement calorifuge 38 peut être assemblé à l'orthèse 30 par couture 43 ou collage.

Un revêtement textile 39 est avantageusement placé de part et d'autre de l'orthèse 30. Le revêtement textile 39 est appliqué contre le revêtement calorifuge 38 d'un côté et contre la première couche 312 de la première enveloppe 31 de l'autre côté. Ledit revêtement textile 39 permet également d'assurer l'imperméabilité et la résistance mécanique dans le temps de l'orthèse 30. Le revêtement textile 39 est préférentiellement assemblé à l'orthèse 30 par couture 43. Le revêtement textile peut être un textile tissé, ou encore une toile en microfibres.

Sur la figure 2 la première couche 312 peut être seulement étanche au liquide, la deuxième couche 313 doit être étanche à la fois au liquide et au gaz, la troisième couche 332 et la quatrième couche 333 peuvent être seulement étanches au gaz.

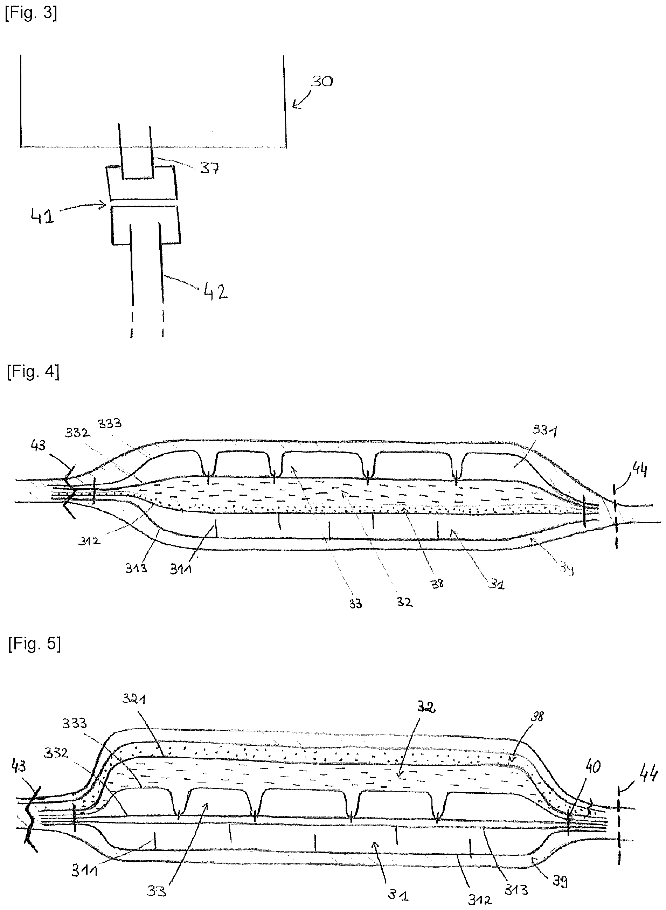

La figure 3 schématise le raccordement d'une des valves 37 à un flexible 42. Seul le raccordement de la valve 37 est représenté sur cette figure, mais la description suivante s'applique à chaque valve 34, 35, 36, 37 de l'orthèse 30.

Lesdites valves 34, 35, 36, 37 sont alimentées en gaz ou en liquide par des flexibles 42. Chaque flexible 42 est relié à l'une desdites valves 34, 35, 36, 37 par l'intermédiaire d'un raccord démontable 41. Ce dernier est préférentiellement un coupleur en laiton nickelé. L'avantage d'utiliser un coupleur en laiton nickelé est d'éviter les fuites de gaz ou de liquide lorsque celui-ci est mis sous pression.

La figure 4 est une vue en coupe de l'orthèse 30 selon un deuxième mode de réalisation.

Dans ce mode de réalisation, le revêtement calorifuge 38 est inséré directement contre la deuxième couche 313 de la première enveloppe 31. Le revêtement calorifuge 38 est assemblé lors de la soudure 40 de la première enveloppe 31 et de la troisième enveloppe 33. Le fait de placer le revêtement calorifuge 38 a cet endroit permet d'obtenir un meilleur rendement du traitement par cryothérapie en limitant davantage les déperditions de chaleur dans l'orthèse 30.

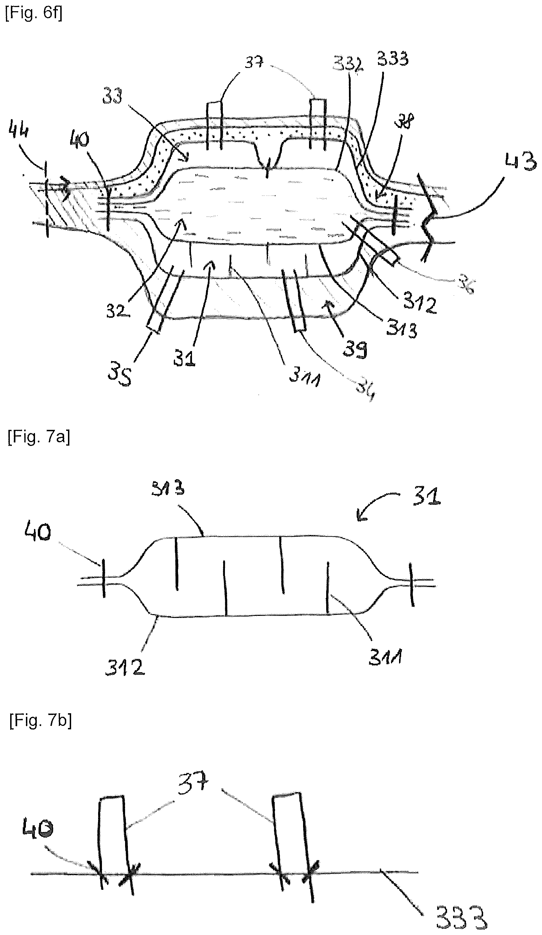

La figure 5 est une vue en coupe de l'orthèse 30 selon un troisième mode de réalisation. Dans ce mode de réalisation, l'orthèse 30 comprend une cinquième couche 321 et dont le procédé de fabrication sera décrit en référence aux figures 7a à 7f.

La première enveloppe 31 est formée par soudure 40 des bords de la première couche 312 et d'une deuxième couche 313. Une troisième enveloppe 33 est formée par soudure 40 d'une troisième couche 332 et d'une quatrième couche 333. La première enveloppe 31 et la troisième enveloppe 33 sont assemblées par soudure 40. Dans ce mode de réalisation, l'espace entre la première enveloppe 31 et la troisième enveloppe 33 reste vide. Une cinquième couche 321 est assemblée à la première enveloppe 31 et la troisième enveloppe 33. Préférentiellement, la cinquième couche 321 et assemblée en regard de la quatrième couche 333 de sorte que la cinquième couche 321 et la quatrième couche 333 forment la poche unique de la deuxième enveloppe 32. Le revêtement calorifuge 38 et le revêtement textile 39 viennent successivement recouvrir la cinquième couche 321.

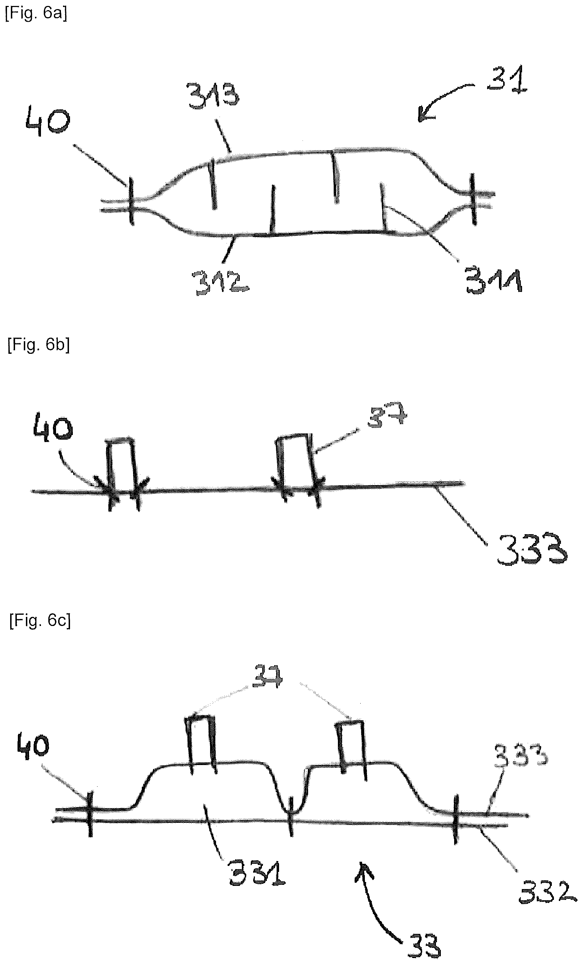

Les figures 6a à 6f illustrent différentes étapes d'un procédé de fabrication de l'orthèse 30 selon le premier mode de réalisation illustré sur la figure 2.

Sur la figure 6a, on assemble les bords de la première couche 312 et la deuxième couche 313 pour former la poche de la première enveloppe 31. L'assemblage de ces dites couches 312, 313 est réalisé préférentiellement par soudure haute fréquence. La soudure haute fréquence permet d'obtenir des soudures étanches résistantes au liquide sous pression circulant dans la première enveloppe 31. La soudure haute fréquence est de meilleure qualité que d'autres techniques connues et permet une meilleure résistance mécanique dans le temps. Un autre avantage de cette technique est qu'elle permet de facilement assembler plusieurs couches ensemble. Lors de l'assemblage de la première couche 312 et de la deuxième couche 313, le circuit hydraulique 311 pour la circulation du fluide est également réalisé par soudure 40 de la première couche 312 et de la deuxième couche 313.

Sur la figure 6b, on assemble les valves d'entrée 37 du gaz sur la quatrième couche 333. Cette étape a pour but de faciliter la mise en place des valves d'entrée 37, de permettre une meilleure fixation de celles-ci qui sera plus stable et plus durable dans le temps. L'assemblage des valves d'entrée 37 sur la quatrième couche 333 est préférentiellement réalisé par soudure haute fréquence ou collage. La présence de plusieurs valves 37 permet de remplir d'air chaque poche 311 de manière indépendante assurant ainsi la fonction de drainage. Pour simplifier la conception, l'assemblage des valves d'entrée 37 sur la quatrième couche 333 est réalisé avant l'assemblage de la troisième couche 332 et de la quatrième couche 333 pour former la troisième enveloppe 33 et la segmentation, représentés à la figure 6c. La segmentation permet de créer les poches 331 indépendantes permettant la compression ou la décompression locale et séquencée du membre traité.

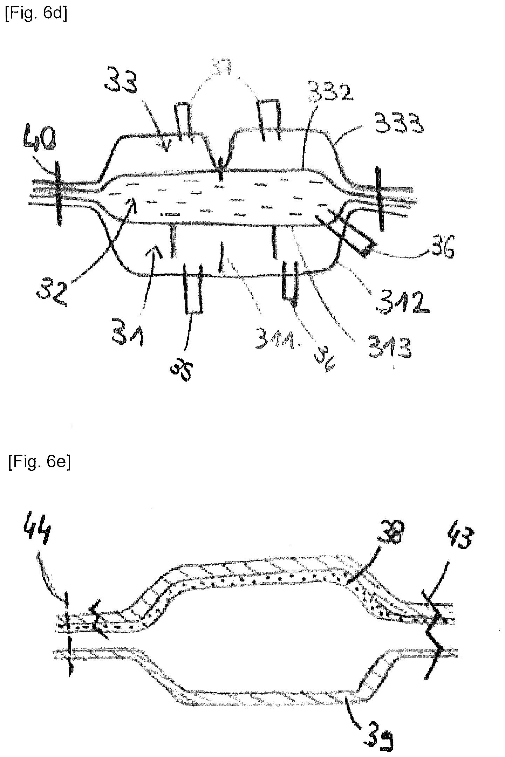

Sur la figure 6d, la première enveloppe 31 est assemblée à la troisième enveloppe 33 pour former la deuxième enveloppe 32 de sorte que la deuxième couche 313 et la troisième couche 332 délimitent la poche unique de ladite deuxième enveloppe 32. L'assemblage de la première enveloppe 31 et de la troisième enveloppe 33 se fait par soudure 40 des quatre couches 312, 313, 332, 333. Cet assemblage permet également de maintenir en place les différentes couches 312, 313, 332, 333 tout en assurant l'étanchéité au gaz et à l'eau. Cet assemblage augmente avantageusement la résistance de l'ensemble de l'orthèse 30. Une fois cet assemblage réalisé, la valve d'entrée 34 hydraulique, la valve de sortie hydraulique 35 et la valve pneumatique 36 d'entrée et de sortie de l'air sont soudées sur leurs enveloppes respectives 31, 32.

Sur la figure 6e, le revêtement calorifuge 38 est assemblé sur la quatrième couche 333 par couture 43, de manière à créer une barrière thermique limitant les déperditions de chaleur et assurant un meilleur rendement du traitement par cryothérapie. Le revêtement textile 39 est ensuite assemblé par couture 43 de manière à recouvrir l'orthèse 30, lequel revêtement recouvre la couche calorifuge 38 et la première couche 312 afin d'apporter à ladite orthèse 30 une meilleure résistance mécanique au cours du temps. Ce procédé de fabrication n'est pas limitatif et peut être adapté selon les différents modes de réalisation décrits dans la demande.

La figure 6f et une représentation de l'enveloppe finale.

Une dernière étape non représentée sur la figure 6 correspond à la découpe finale de l'orthèse à une distance non limitative de 5mm de la soudure 40. Cette découpe est préférentiellement faite à la main à l'aide d'une paire de ciseaux.

Les figures 7a à 7g illustrent différentes étapes d'un procédé de fabrication de l'orthèse 30 selon le troisième mode de réalisation illustré sur la figure 5.

Les étapes 7a à 7c sont identiques au procédé de réalisation décrit aux figures 6a à 6c.

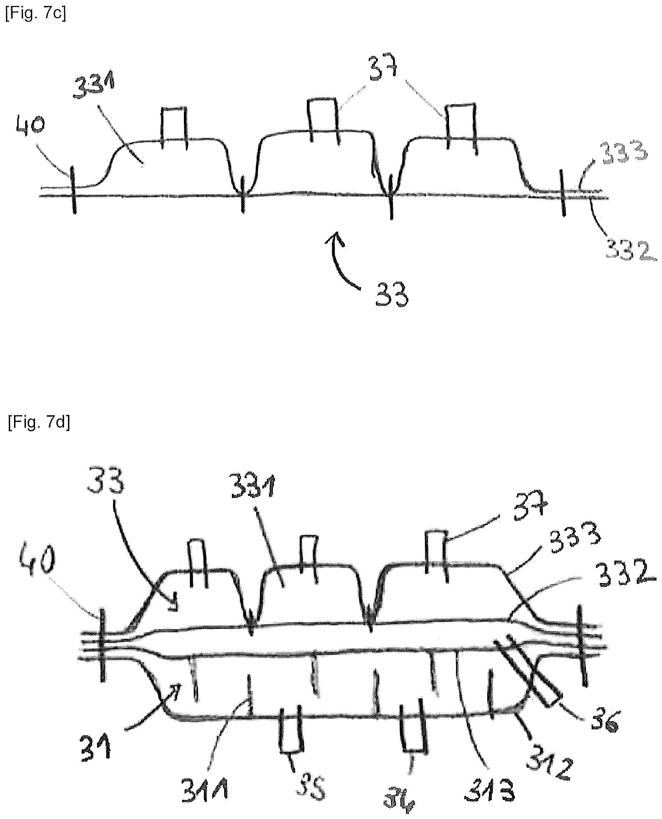

Sur la figure 7d, la première enveloppe 31 est assemblée à la troisième enveloppe 33 par soudure 40. Une fois cet assemblage réalisé, la valve d'entrée 34 hydraulique, la valve de sortie hydraulique 35 et la valve pneumatique 36 d'entrée et de sortie de l'air sont soudées sur leurs enveloppes respectives 31, 32.

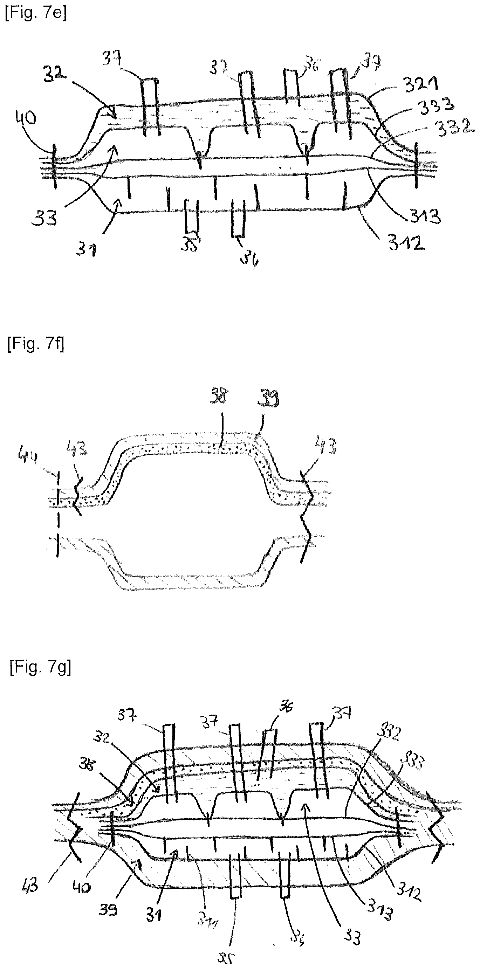

Sur la figure 7e, une cinquième couche 321 est assemblée par soudure 40 à la quatrième couche 333, de telle sorte que la cinquième couche 321 et la quatrième couche 333 forment la deuxième enveloppe 32.

L'étape illustrée sur la figure 7f est identique à celle décrite en référence à la figure 6e.

La figure 7g et une représentation de l'enveloppe finale.

Une dernière étape non représentée sur la figure 7 correspond à la découpe finale de l'orthèse à une distance non limitative de 5mm de la soudure 40. Cette découpe est préférentiellement faite à la main à l'aide d'une paire de ciseaux.

Comparé au procédé de fabrication décrit en référence aux figures 6a à 6e, ce troisième mode de réalisation et son procédé de fabrication associé ne sont pas optimaux puisqu'ils sont plus longs, plus couteux (car on ajoute une cinquième couche 321 contre quatre couches 312, 313, 332, 333 dans les deux autres modes de réalisation décrits précédemment) et nécessitent une étape supplémentaire d'assemblage de la cinquième couche 321 pour la fabrication de l'orthèse 30.

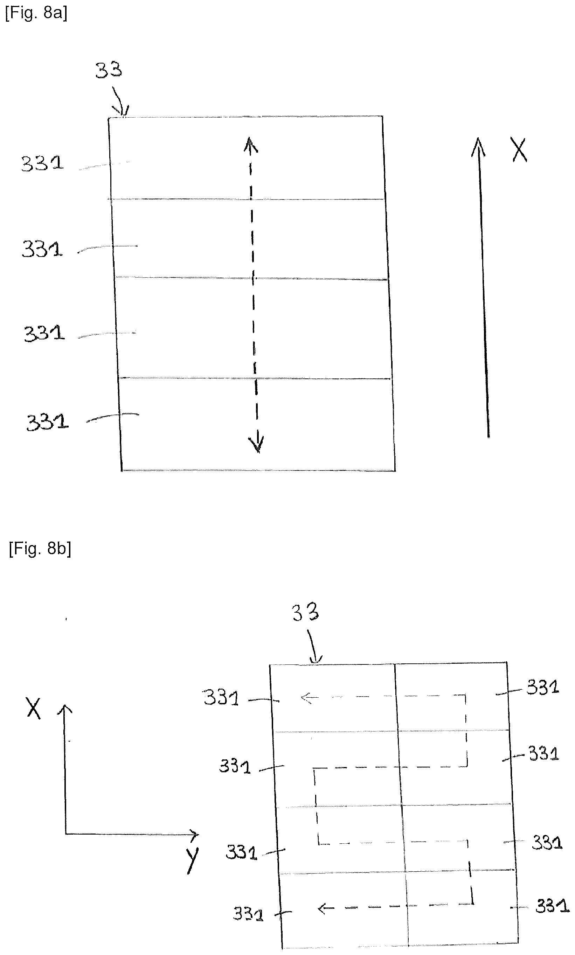

Les figures 8a et 8b représentent deux modes de réalisation de la troisième enveloppe 33 et du déplacement de la vague lors du drainage.

Sur la figure 8a, la troisième enveloppe comporte une série de poches alignées selon l'axe X. Le cheminement de la vague se fait selon cet axe X.

Sur la figure 8b, la troisième enveloppe 33 comporte deux séries (ou plus) parallèles de poches, lesquelles séries sont décalées l'une de l'autre selon l'axe Y, et les poches de chaque série étant alignées selon l'axe X. Dans cette configuration, on peut faire serpenter le cheminement de la vague selon les axes X et Y.

L'orthèse 30 peut être utilisée avec une hipposandale 60 adaptée pour le traitement des membres des ongulés. Les hipposandales sont connues pour la protection des sabots, notamment pour ne pas abimer la corne et remplacer le ferrage. Les hipposandales sont également utilisées pour diminuer la fréquence des affections articulaires notamment avec l'utilisation de semelles en caoutchouc atténuant les chocs. Certains brevets existent dans le domaine, notamment le document WO2014/138790 (Macdonald) mais qui n'est toutefois pas adapté pour le traitement des sabots et des articulations car l'hipposandale décrite dans ce document n'assure qu'une protection du sabot et non pas un traitement de ce dernier.

Le traitement des ongulés peut être amélioré en associant l'orthèse 30 avec une hipposandale 60 adaptée pour le traitement cryogénique des sabots.

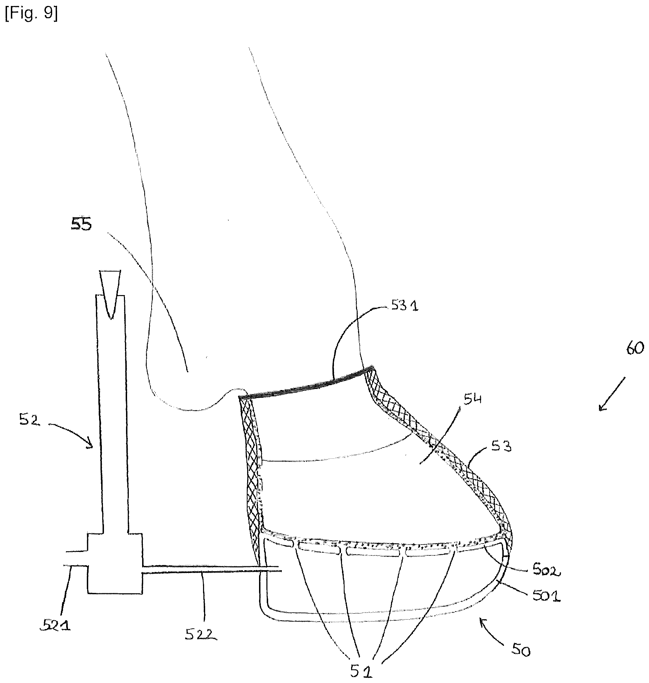

La figure 9 représente une hipposandale selon l'invention se composant d'une semelle cryogénique 50 sur laquelle repose un sabot 54 et d'une membrane textile 53. La semelle cryogénique 50 assure un traitement par cryothérapie des sabots d'ongulé en créant une sensation de froid pour soulager les articulations. La semelle cryogénique 50 se compose d'une demi-coquille inférieure 501 et d'une demi-coquille supérieure 502 formant une cavité creuse. Les semelles cryogéniques 50 sont fabriquées dans une matière synthétique très résistante telle que du polyuréthane thermoplastique. Les demi-coquilles inférieure 501 et supérieure 502 forment une chambre étanche. La semelle cryogénique est couplée à un tube vortex 52. Ce dernier convertit un gaz sous pression en deux courants d'air, un courant d'air chaud et un courant d'air froid. Le gaz arrive dans le tube vortex 52 par un tube d'arrivée de gaz sous pression 521. Préférentiellement, le courant d'air froid est sélectionné pour être injecté dans la semelle cryogénique 50 au travers d'un tube de sortie du gaz 522. L'utilisation du tube vortex a pour avantage d'être facile à mettre en oeuvre et d'être peu coûteuse. Un autre avantage d'utiliser un tube vortex est qu'il ne requiert aucune maintenance, sa durée de vie est donc longue. Le tube vortex utilisé est par exemple commercialisé par la société Albret. En cas de besoin, l'homme du métier pourra se référer à la page internet http://albret-thermic.com/ pour davantage de précisions sur le fonctionnement d'un tube vortex.

La semelle cryogénique 50 est combinée à la membrane textile 53 pour former une sorte de chaussette dans laquelle s'insère le sabot 54. Ledit textile 53 est étanche à l'air de tel sort que le gaz froid reste au contact du sabot. Ledit textile 53 a également pour fonction de maintenir la semelle cryogénique 50 en place sous le sabot. Le textile 53 est pourvu d'un élastique 531. L'élastique 531 maintient en place le textile 53 au niveau du boulet 55.

La demi-coquille supérieure 502 et pourvue de perforations 51.

Le gaz froid sortant du tube vortex par le tube de sortie 522 se dirige dans la semelle cryogénique 50 puis à travers les perforations 51 de la demi-coquille supérieure 502 pour venir autour du sabot 54. L'élastique 531 permet de rendre étanche la chaussette formée par le textile 53 tout en évitant les déperditions de chaleur. Le gaz froid peut donc circuler autour du sabot en abaissant la température pour créer une sensation de froid soulageant les articulations.

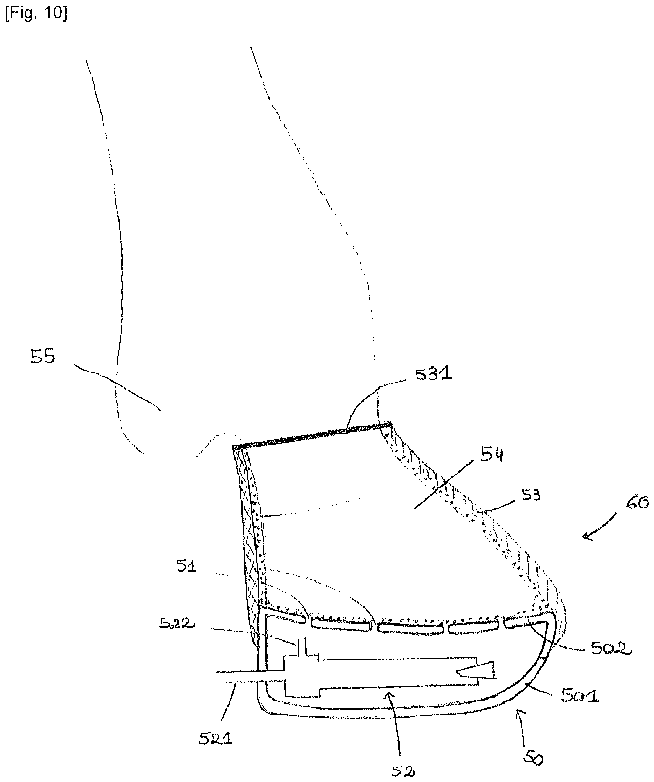

La figure 10 représente une hipposandale selon l'invention selon un second mode de réalisation dans lequel le tube vortex 52 est disposé dans la semelle cryogénique 50. Le mode de fonctionnement est le même que celui présenté à la figure 9.

Une ou plusieurs caractéristiques exposées seulement dans un mode de réalisation peuvent être combinées avec une ou plusieurs autres caractéristiques exposées seulement dans un autre mode de réalisation. L'agencement des différents éléments et/ou moyens et/ou étapes de l'invention, dans les modes de réalisation décrits ci-dessus, ne doit pas être compris comme exigeant un tel agencement dans toutes les implémentations. Notamment :
- Le liquide utilisé n'est pas nécessairement de l'eau déminéralisée mélangée à un antigel, il est possible d'utiliser un mélange d'eau avec de l'huile. L'antigel peut être du type éthylène glycol ou propylène glycol.
- Les couches 332, 333, 312, 313 de l'orthèse 30 n'ont pas nécessairement la même épaisseur, celle-ci pouvant varier d'une enveloppe à l'autre et/ou d'une couche à l'autre.
- Le raccord démontable 41 n'est pas nécessairement un coupleur en laiton nickelé, il est possible également d'utiliser un raccord en matière plastique ou encore un raccord en cuivre.
- Le revêtement calorifuge 38 n'est pas nécessairement constitué d'une couche d'aluminium, il est possible d'utiliser un film PET (polytéréphtalate d'éthylène).
- Les différentes soudures 40 ne sont pas nécessairement obtenues par soudure haute fréquence, il est possible également de réaliser ces soudures par thermo-soudage ou des soudures à impulsion.
- La cinquième couche 321 n'est pas nécessairement assemblée en regard de la quatrième couche 333. Il est possible également d'assembler la cinquième couche 321 en regard de la première couche 312, de sorte que la cinquième couche 321 et la première couche 312 forment le compartiment unique de la deuxième enveloppe 32.
- Le courant d'air froid n'est pas nécessairement le seul gaz qui peut être injecté dans la semelle cryogénique 50, il est également possible d'injecter le gaz chaud dans la semelle cryogénique. Il est également possible d'injecter alternativement l'air chaud et l'air froid. L'alternance des phases d'envoi d'air chaud et d'air froid permet d'améliorer et d'adapter le traitement.
- La découpe de l'orthèse n'est pas nécessairement faire à la main à l'aide d'une paire de ciseaux, il est également possible d'utiliser un outillage de découpe mécanique.

En outre, une ou plusieurs caractéristiques exposées seulement dans un mode de réalisation peuvent être combinées avec une ou plusieurs autres caractéristiques exposées seulement dans un autre mode de réalisation. De même, une ou plusieurs caractéristiques exposées seulement dans un mode de réalisation peuvent être généralisées aux autres modes de réalisation.

## Revendications

1. [Orthèse (30) adaptée pour un traitement par cryothérapie et/ou pressothérapie d'une zone d'un corps humain ou animal comportant :
- une première enveloppe (31) adaptée pour un traitement par cryothérapie, laquelle première enveloppe (31) comporte une poche hydraulique adaptée pour recevoir un liquide,
- une deuxième enveloppe (32) adaptée pour un traitement par pressothérapie avec effet de pompage, laquelle deuxième enveloppe (32) comporte une seule poche gonflable,
**et caractérisée en ce qu'elle comporte** :
- une troisième enveloppe (33) adaptée pour un traitement par pressothérapie avec effet de drainage, laquelle troisième enveloppe (33) comporte plusieurs poches (331) gonflables indépendantes.

2. Orthèse (30) selon la revendication 1, dans laquelle la première enveloppe (31) est formée d'une première couche (312) et d'une deuxième couche (313) assemblées par soudure (40) de leurs bords.

3. Orthèse (30) selon la revendication 1, dans laquelle la troisième enveloppe (33) est formée d'une troisième (332) et d'une quatrième couche (333) assemblées par soudure (40) de leurs bords.

4. Orthèse (30) selon la revendication 1, dans laquelle la première enveloppe (31) et la troisième enveloppe (33) sont assemblées par soudure (40).

5. Orthèse (30) selon la revendication 1, dans laquelle la deuxième enveloppe (32) est formée par soudure de la première enveloppe (31) et de la troisième enveloppe (33).

6. Orthèse (30) selon la revendication 3, dans laquelle les poches (331) indépendantes sont formées par soudure (40) de la troisième couche (332) et de la quatrième couche (333).

7. Orthèse (30) selon la revendication 1, dans laquelle chaque poche (331) indépendante est reliée à une valve (37).

8. Orthèse (30) selon la revendication 1, comportant un revêtement textile (39).

9. Orthèse (30) selon la revendication 8, dans laquelle un revêtement calorifuge (38) est disposé entre le revêtement textile (39) et la troisième enveloppe (33).

10. Procédé de fabrication d'une orthèse (30) conforme à la revendication 1, comportant les étapes consistant à :
- former la première enveloppe (31) en assemblant une première couche (312) et une deuxième couche (313), de manière à délimiter la poche hydraulique,
- former la troisième enveloppe (33) en assemblant une troisième couche (332) et une quatrième couche (333), de manière à délimiter les poches (331) gonflables indépendantes,
- former la deuxième enveloppe (32) en assemblant la première enveloppe (31) et la troisième enveloppe (33) de sorte que la deuxième couche (313) et la troisième couche (332) délimitent la poche de ladite deuxième enveloppe.

11. Procédé de fabrication de l'orthèse (30) selon la revendication 10 , dans lequel un revêtement calorifuge (38) est cousu sur la quatrième couche (333).

12. Procédé de fabrication de l'orthèse (30) selon la revendication 11, dans lequel un revêtement textile (39) vient recouvrir le revêtement calorifuge (38).

13. Procédé de fabrication de l'orthèse (30) selon la revendication 12, dans lequel le revêtement textile (39) vient recouvrir le première couche (312).

14. Ensemble comportant l'orthèse 30 conforme à la l'une des revendications précédentes et une hipposandale (60) pour le traitement des pattes d'animaux, ladite hipposandale comprenant une semelle cryogénique (50), une partie textile (53) et un tube vortex (52).

15. Ensemble selon la revendication 14 dans lequel la semelle cryogénique (50) comporte une demi-coquille inférieure (501) et une demi-coquille supérieure (502).

16. Ensemble selon la revendication 15, dans lequel la demi-coquille supérieure (502) comporte au moins une perforation (51).

17. Ensemble selon l'une des revendications 14 à 16, dans lequel le tube vortex (52) est relié à la semelle cryogénique (50).

18. Ensemble selon l'une des revendications 15 à 17, dans lequel le textile (53) est relié à la semelle cryogénique (50) de manière à pouvoir envelopper un sabot (54).

## Patentansprüche

1. Orthese (30), die für eine Behandlung durch Kryotherapie und/oder Drucktherapie eines Bereichs eines menschlichen oder tierischen Körpers geeignet ist, umfasst: - eine erste Hülle (31), die für eine Kryotherapie geeignet ist, wobei die erste Hülle (31) einen Hydraulikbeutel aufweist, der zur Aufnahme einer Flüssigkeit geeignet ist,
- eine zweite Hülle (32), die für eine Drucktherapie mit Pumpeffekt geeignet ist, wobei die zweite Hülle (32) einen aufblasbaren Beutel aufweist,
**und dadurch gekennzeichnet ist, dass sie den folgenden Teil umfasst:**
- eine dritte Hülle (33), die für eine Drucktherapie mit Drainageeffekt geeignet ist, wobei die dritte Hülle (33) mehrere unabhängige aufblasbare Beutel (331) aufweist.

2. Orthese (30) nach Anspruch 1, wobei die erste Hülle (31) aus einer ersten Schicht (312) und einer zweiten Schicht (313) besteht, die durch Verschweißen (40) ihrer Ränder zusammengefügt sind.

3. Orthese (30) nach Anspruch 1, wobei die dritte Hülle (33) aus einer dritten Schicht (332) und einer vierten Schicht (333) besteht, die durch Verschweißen (40) ihrer Ränder zusammengefügt sind.

4. Orthese (30) nach Anspruch 1, wobei die erste Hülle (31) und die dritte Hülle (33) durch Verschweißen (40) zusammengefügt sind.

5. Orthese (30) nach Anspruch 1, wobei die zweite Hülle (32) durch Verschweißen der ersten Hülle (31) und der dritten Hülle (33) gebildet wird.

6. Orthese (30) nach Anspruch 3, wobei die unabhängigen Beutel (331) durch Verschweißen (40) der dritten Schicht (332) und der vierten Schicht (333) gebildet werden.

7. Orthese (30) nach Anspruch 1, wobei jeder unabhängige Beutel (331) mit einem Ventil (37) verbunden ist.

8. Orthese (30) nach Anspruch 1, mit einer Textilbeschichtung (39).

9. Orthese (30) nach Anspruch 8, wobei sich eine wärmeisolierende Beschichtung (38) zwischen der Textilbeschichtung (39) und der dritten Hülle (33) befindet.

10. Das Verfahren zur Herstellung einer Orthese (30) nach Anspruch 1 umfasst folgende Schritte:
- Erstellen der ersten Hülle (31) durch Zusammenfügen einer ersten Schicht (312) und einer zweiten Schicht (313), um so den Hydraulikbeutel abzugrenzen,
- Erstellen der dritten Hülle (33) durch Zusammenfügen einer dritten Schicht (332) und einer vierten Schicht (333), um so die unabhängigen aufblasbaren Hydraulikbeutel (331) abzugrenzen,
- Erstellen der zweiten Hülle (32) durch Zusammenfügen der ersten Hülle (31) und der dritten Hülle (33), so dass die zweite Schicht (313) und die dritte Schicht (332) den Beutel der zweiten Hülle abgrenzen.

11. Verfahren zur Herstellung der Orthese (30) nach Anspruch 10, wobei eine wärmeisolierende Beschichtung (38) auf die vierte Schicht (333) genäht wird.

12. Verfahren zur Herstellung der Orthese (30) nach Anspruch 11, wobei eine Textilbeschichtung (39) die wärmeisolierende Beschichtung (38) abdeckt.

13. Verfahren zur Herstellung der Orthese (30) nach Anspruch 12, wobei eine Textilbeschichtung (39) die erste Schicht (312) abdeckt.

14. Set mit Orthese 30 gemäß einem der vorhergehenden Ansprüche und einer Hipposandale (60) zur Behandlung von Tierpfoten, wobei die Hipposandale eine Kühlsohle (50), einen Textilteil (53) und ein Wirbelrohr (52) umfasst.

15. Set nach Anspruch 14, wobei die Kühlsohle (50) eine untere Halbschale (501) und eine obere Halbschale (502) aufweist.

16. Set nach Anspruch 15, wobei die halbe-Oberschale (502) mindestens eine Perforation (51) aufweist.

17. Set nach einem der Ansprüche 14 bis 16, wobei das Wirbelrohr (52) mit der Kühlsohle (50) verbunden ist.

18. Set nach einem der Ansprüche 15 bis 17, wobei das Textilteil (53) so mit der Kühlsohle (50) verbunden ist, dass es einen Huf (54) umhüllen kann.

## Claims

1. Orthosis (30) adapted for cryotherapy and/or pressotherapy treatment of an area of a human or animal body comprising: -a first envelope (31) adapted for cryotherapy treatment, which first envelope (31) comprises a hydraulic pocket adapted to receive a liquid,
- a second envelope (32) adapted for pressotherapy treatment with pumping effect, which second envelope (32) comprises a single inflatable pocket,
**and characterized in that it comprises:**
- a third envelope (33) suitable for pressotherapy treatment with drainage effect, which third envelope (33) comprises several independent inflatable pockets (331).

2. .Orthosis (30) according to claim 1, in which the first envelope (31) comprises a first layer (312) and a second layer (313) assembled by welding (40) their edges.

3. Orthosis (30) according to claim 1, in which the third envelope (33) comprises a third layer (332) and a fourth layer (333) assembled by welding (40) their edges.

4. Orthosis (30) according to claim 1, in which the first envelope (31) and the third envelope (33) are assembled by welding (40).

5. Orthosis (30) according to claim 1, in which the second envelope (32) is formed by welding the first envelope (31) and the third envelope (33).

6. Orthosis (30) according to claim 3, in which the independent pockets (331) are formed by welding (40) the third layer (332) and the fourth layer (333).

7. Orthosis (30) according to claim 1, in which each independent pocket (331) is connected to a valve (37).

8. Orthosis (30) according to claim 1, comprising a textile covering (39).

9. Orthosis (30) according to claim 8, in which a heat-insulating covering (38) is arranged between the textile covering (39) and the third envelope (33).

10. Method of manufacturing an orthosis (30) according to claim 1, comprising the steps of:
- forming the first envelope (31) by assembling a first layer (312) and a second layer (313), so as to define the hydraulic pocket,
- forming the third envelope (33) by assembling a third layer (332) and a fourth layer (333), so as to define the independent inflatable pockets (331),
- forming the second envelope (32) by assembling the first envelope (31) and the third envelope (33) so that the second layer (313) and the third layer (332) define the pocket of said second envelope.

11. Method of manufacturing the orthosis (30) according to claim 10, in which a heat-insulating covering (38) is sewn onto the fourth layer (333).

12. Method of manufacturing the orthosis (30) according to claim 11, in which a textile covering (39) covers the heat-insulating covering (38).

13. Method of manufacturing the orthosis (30) according to claim 12, in which a textile covering (39) covers the heat-insulating covering (312).

14. Assembly comprising orthosis 30 according to one of the preceding claims and a hipposandal (60) for the treatment of animal paws, with said hipposandal comprising a cryogenic sole (50), a textile part (53) and a vortex tube (52).

15. Assembly according to claim 14, in which the cryogenic sole (50) comprises a lower half-shell (501) and an upper half-shell (502).

16. Assembly according to claim 15, in which the upper half-shell (502) comprises at least one perforation (51).

17. Assembly according to one of claims 14 to 16, in which the vortex tube (52) is connected to the cryogenic sole (50).

18. Assembly according to one of claims 15 to 17, in which the textile (53) is connected to the cryogenic sole (50) so as to be able to cover a hoof (54).
